# EUROPEAN PATENT SPECIFICATION

(11) **EP 3 260 064 B1**
(45) Date of publication and mention of the grant of the patent: **18.11.2020**
(21) Application number: 16783035.5
(22) Date of filing: 08.04.2016
(51) Int. Cl.: A61B 17/32, A61B 17/16, A61B 17/56

(54) **TREATMENT DEVICE AND TREATMENT SYSTEM**
BEHANDLUNGSVORRICHTUNG UND BEHANDLUNGSSYSTEM
DISPOSITIF DE TRAITEMENT ET SYSTÈME DE TRAITEMENT

(30) Priority: 22.04.2015 JP 2015087631
(43) Date of publication of application: 27.12.2017
(73) Proprietor: Olympus Corporation, Hachioji-shi, Tokyo 192-8507 (JP)
(72) Inventor: ONUMA, Chie, Hachioji-shi, Tokyo 192-8507 (JP); SAKAI, Masahiro, Hachioji-shi, Tokyo 192-8507 (JP)
(74) Representative: Schicker, Silvia
(86) International application number: PCT/JP2016/061581
(87) International publication number: WO 2016/171014

(56) References cited:
- WO-A1-2008/118709
- WO-A1-2010/087060
- JP-A- 2005 066 079
- JP-A- 2013 031 493
- JP-A- 2013 031 493
- JP-A- 2013 519 441
- JP-A- 2015 051 274
- US-A1- 2002 077 550
- US-A1- 2005 245 823

## Description

### FIELD

The present invention relates to a treatment device that is capable of performing treatment such as incision and excision relative to living tissue.

### BACKGROUND

Jpn. Pat. Appln. KOKAI Publication No. 2003-116870 discloses an ultrasonic handpiece used for excision of hard tissue such as a bone, for example. The ultrasonic handpiece includes an ultrasonic probe having a surgical knife portion at the distal end, an opening portion provided in the ultrasonic probe close to the surgical knife portion, a suction path provided inside the ultrasonic probe that communicates with the opening portion, and a suction mechanism connected to the suction path.

This ultrasonic handpiece can perform suction removal of moisture, excision fragments, splinters, etc. through the opening portion.

US 2005/0245823 A1 refers to an ultrasonic treatment device composed of a handpiece, a probe unit, an inner sheath unit, and an outer sheath unit. In the handpiece, an ultrasonic transducer generating an ultrasonic vibration is incorporated. In the probe unit, a long rod-like probe whose base end is connected to the ultrasonic transducer is placed. This probe transmits, toward its distal end, the ultrasonic vibration generated by the transducer. The inner sheath unit includes a tubular inner sheath to cover the probe. The outer sheath unit includes an outer sheath to cover the inner sheath. To the distal end of the probe is secured a treatment piece. The inner sheath has a distal end on which a cylindrical protective member is arranged to protect the distal end of the probe. The outer sheath unit is provided with a large-diameter connecter disposed at a base end of the outer sheath. The connecter has a substantially cylindrical connecting main body and a substantially cylindrical rubber member. The outer sheath is sustained by the rubber member coaxially with the inner sheath, resulting in that an annular and cylindrical sheath-to-sheath suction path is formed between the inner and outer sheaths.

JP 2013 031493 A relates to a fluid jetting device which comprises a grasping part, a sucking tube protrudingly arranged in the grasping part, a jetting tube eccentrically inserted into the sucking tube such that an outer peripheral surface comes into contact with an inner surface of the sucking tube and having a jetting opening part protrudingly arranged to in the grasping part, a bubble generating member, a sucking flowing channel and a sucking opening part formed between the inner peripheral surface of the sucking tube and the outer peripheral surface of the jetting tube. The jetting tube is fixed to the inner peripheral surface of the sucking tube in the vicinity of the jetting opening part.

### CITATION LIST

### PATENT LITERATURE

Patent Literature 1: Jpn. Pat. Appln. KOKAI Publication No. 2003-116870

### TECHNICAL PROBLEM

However, when performing suction removal through an opening portion or a suction path provided to an ultrasonic probe such as the aforementioned ultrasonic handpiece, the opening portion or the suction path may be clogged with excision fragments, splinters, etc. If the opening portion or the suction path becomes clogged with excision fragments, splinters, etc., the vibration properties of the ultrasonic probe may be adversely affected. On the other hand, if the diameter of the opening portion or the suction path is formed to be greater to avoid the clogging, a problem in that the strength of the ultrasonic probe cannot be maintained, or that the ultrasonic probe is enlarged may occur.

An object of the present invention is to provide a treatment device that is capable of performing treatment while performing suction removal of treatment fragments with a simple structure.

### SOLUTION TO PROBLEM

The present invention relates to a treatment device as defined in claim 1 and a treatment system as defined in claim 6. Preferred embodiments are defined by the dependent claims. To achieve the above object, according to an aspect of the present disclosure, a treatment device may include a probe having a treatment portion that performs treatment to a treatment target portion, ultrasonic vibration being transmitted to the probe; a first sheath having a first edge and covering the periphery of the probe; a second sheath having a second edge, and covering the periphery of the first sheath; a suction path including a suction opening between the first edge and the second edge and provided between the first sheath and the second sheath; and a connection part communicating with the suction path and connected to a suction source.

The treatment system may include a probe having a treatment portion that performs treatment to a treatment target portion, ultrasonic vibration being transmitted to the probe; a first sheath having a first edge and covering the periphery of the probe; a second sheath having a second edge and covering the periphery of the first sheath; a suction path including a suction opening between the first edge and the second edge and provided between the first sheath and the second sheath; and a suction source provided to be connected to the suction path.

### ADVANTAGEOUS EFFECTS OF INVENTION

The present invention can provide a treatment device that is capable of performing treatment while performing suction removal of treatment fragments with a simple structure.

### BRIEF DESCRIPTION OF THE DRAWINGS

FIG. 1 is a schematic diagram illustrating a treatment system for a joint according to the first embodiment.
FIG. 2 is a schematic cross-sectional view of an enlarged probe, first sheath and second sheath of a treatment device of the treatment system depicted in FIG. 1.
FIG. 3 is a schematic cross-sectional view of a vibration generator of the treatment system depicted in FIG. 1.
FIG. 4A is a cross-sectional view of a probe, a first sheath, a second sheath, and a suction path of a treatment system for a joint according to a first example useful to understand the present invention.
FIG. 4B is a schematic diagram of an enlarged treatment portion of the probe depicted in FIG. 4A.
FIG. 5 is a schematic diagram of the probe and the suction path depicted in FIG. 4 in which the height H1 of the probe in the direction crossing the longitudinal direction of the probe and the height H2 of the suction path are indicated.
FIG. 6A is a cross-sectional view of a probe, a first sheath, a second sheath, and a suction path of a treatment system for a joint according to a modification of the first example.
FIG. 6B is a cross-sectional view taken along line B-B of the probe depicted in FIG. 6A.
FIG. 7 is a schematic cross-sectional view of an enlarged probe, first sheath, second sheath, suction path, and opening portion of a treatment device of a treatment system for a joint according to a second example useful to understand the present invention.
FIG. 8 is a schematic cross-sectional view of an enlarged probe, second sheath, and a plurality of opening portions of a treatment device of a treatment system according to a first modification of the second example.
FIG. 9 is a schematic cross-sectional view of an enlarged probe, second sheath, and opening portion of a treatment device of a treatment system according to a second modification of the second example.
FIG. 10 is a schematic cross-sectional view of an enlarged probe, first sheath, second sheath, and suction path of a treatment device of a treatment system for a joint according to a second embodiment.
FIG. 11 is a front view of the treatment device when viewed from an arrow A depicted in FIG. 10.
FIG. 12 is a schematic cross-sectional view of an enlarged probe, first sheath, second sheath, and suction path of a treatment device of a treatment system for a joint according to a third embodiment.
FIG. 13 is a front view of the treatment device when viewed from an arrow A depicted in FIG. 12.

### DETAILED DESCRIPTION

### [First Embodiment]

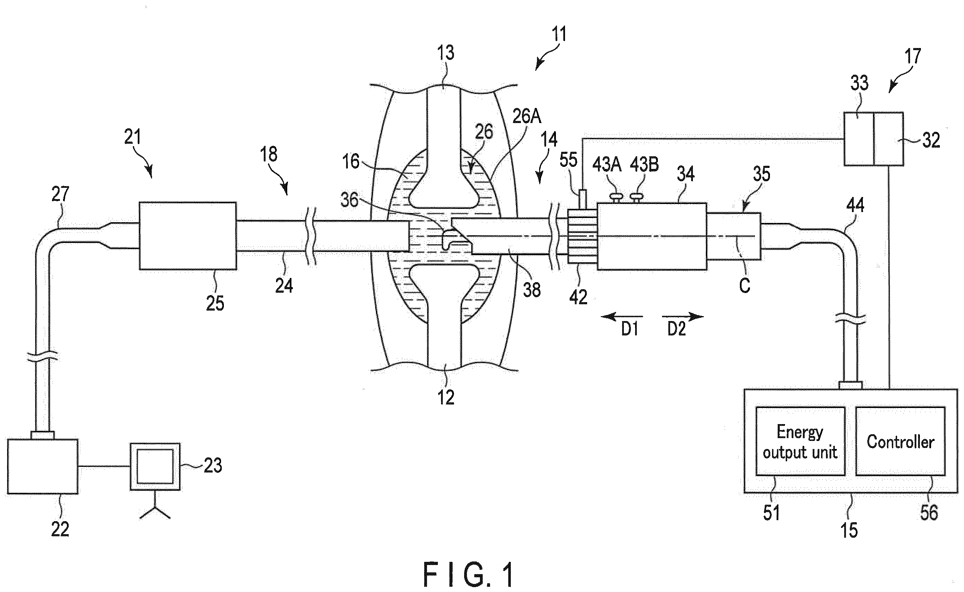

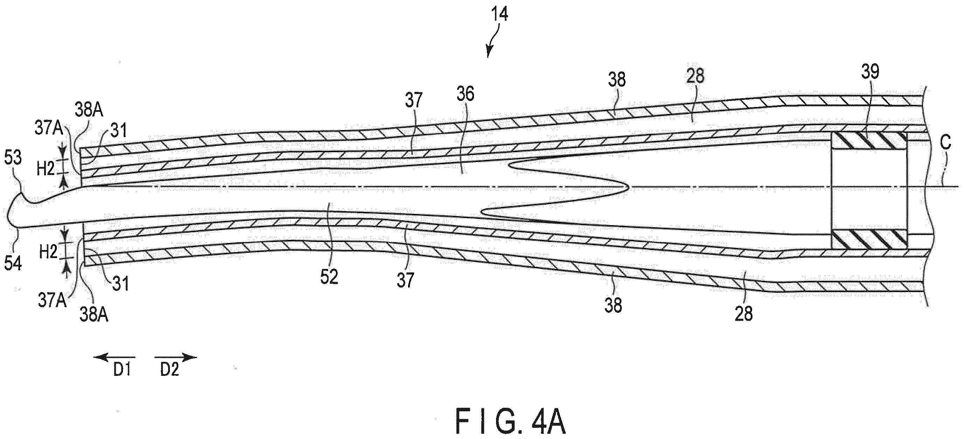

The first embodiment of the present invention will be explained with reference to FIGS. 1 to 3, and a first example useful to understand the present invention will be explained with reference to FIGS. 4A to 5. As shown in FIG. 1, a treatment system 11 is used for treatment within a joint such as a shoulder, knee, elbow, etc., namely, between a first bone 12 and a second bone 13. The treatment system 11 includes a treatment device 14, a power supply unit 15 that acts as a power supply apparatus to drive the treatment device 14, a suction source 17 that applies a suction power to a distal end portion of the treatment device 14 to suction fragments of living tissue subjected to the treatment of the treatment device 14 (living tissue fragments, debris), blood, cavitation foam, perfusate 16 contaminated by them, etc., and an endoscope apparatus 21 that includes an arthroscope 18.

The endoscope apparatus 21 includes the arthroscope 18, an image processing unit 22, and a display portion 23 such as a display.

The arthroscope 18 includes an insertion portion 24 and a holding portion 25. In treatment using the treatment system 11, a distal end portion of the insertion portion 24 is inserted into a joint cavity 26. One end of a universal cord 27 is connected to the holding portion 25. The other end of the universal cord 27 is connected to the image processing unit 22 such as an image processor. The image processing unit 22 is electrically coupled to a display portion 23 such as a monitor.

The insertion portion 24 is provided with an imaging element at a distal end. The imaging element captures an image of a subject through an observation window. The imaging element is electrically coupled to the image processing unit 22 through an imaging cable that passes through the inside of the insertion portion 24, the holding portion 25, and the universal cord 27. The image processing unit 22 performs image processing to the captured subject image to display the image on the display portion 23. The arthroscope 18 is coupled to a light source unit (not shown in the drawings), and light emitted from the light source unit is applied to the subject.

The suction source 17 includes a vacuum pump 32 that is connected to a suction path 28 (connection port) so that a suction power (negative pressure) is applied to a suction opening 31 formed at the distal end of the treatment device 14; and a tank 33 provided closer to the suction path 28 side than the vacuum pump 32, that collects the living tissue fragments processed by the treatment device 14, blood, cavitation foam, and the perfusate 16 contaminated by them.

As shown in FIGS. 1 to 4, the treatment device 14 includes a case 34 which is an outer shell; a vibration generator 35 (transducer) housed within the case 34; a rod-like probe 36 connected to the vibration generator 35; a first hollow (cylindrical) sheath (inner sheath) 37 that covers the periphery of the probe 36 to protect the probe 36; a second hollow (cylindrical) sheath (outer sheath) 38 that covers the periphery of the first sheath 37; an adapter 41 attached to an inner surface of the case 34 to support the second sheath 38; the suction path 28 provided to the outside of the first sheath 37 and the inside of the second sheath 38; a knob (rotation knob) 42 fixed rotatably to the case 34; a plurality of buttons 43A and 43B provided in the case 34; and a sealing member 39 provided between the probe 36 and the first sheath 37. In the following explanation, it is assumed as shown in FIG. 1 that the direction indicated by an arrow D1 is a distal end direction of the probe 36, the direction indicated by an arrow D2 is the proximal end direction of the probe 36, and an axis C is the longitudinal direction (longitudinal axis, central axis) of the probe 36.

As shown in FIG. 1, one end of a cable 44 is coupled to the vibration generator 35. The other end of the cable 44 is connected to the power supply unit 15. The knob 42 is attached to the case 34 rotatably around the central axis C of the probe 36. The knob 42 is coupled to the probe 36 through a coupling mechanism not shown in the drawings. When the knob 42 is rotated relative to the case 34, the probe 36 is integrally rotated around the central axis C.

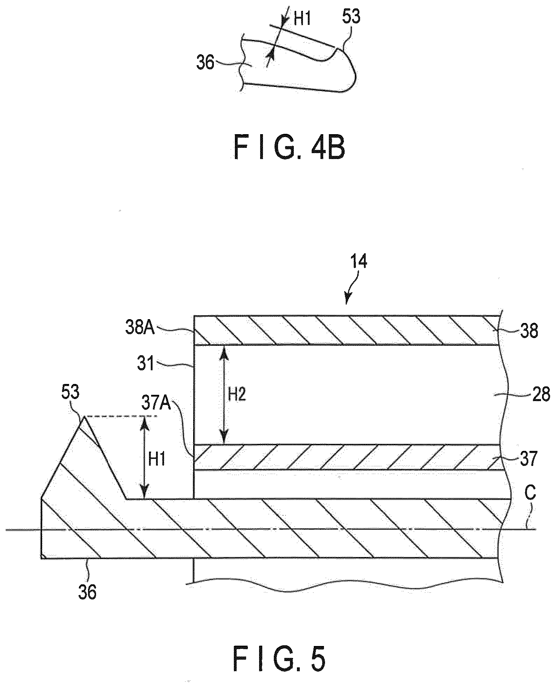

As shown in FIG. 4, the sealing member 39 is arranged at a position of a node of ultrasonic vibrations transmitted to the probe 36, and is used as a spacer to reliably retain a space between the probe 36 and the first sheath 37, so as to prevent liquid from entering the proximal end side of the probe 36. The sealing member 39 is made of a resin (elastic member) having elasticity like a rubber in a ring shape.

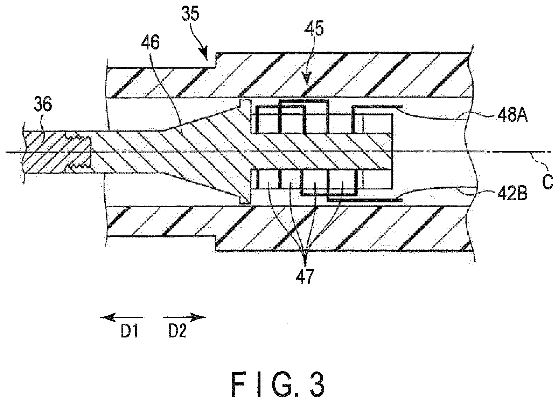

As shown in FIG. 3, the vibration generator 35 includes an ultrasonic vibrator 45 and a horn member 46. The ultrasonic vibrator 45 is provided, for example, with four piezoelectric elements 47 that change power (energy) into ultrasonic vibration. The ultrasonic vibrator 45 is coupled to one end of a first electrical wiring 48 (48A and 48B). The first electrical wiring 48 extends inside the cable 44 and connects with an energy output unit 51 of the power supply unit 15 at the other end. The energy output unit 51 outputs power to drive the ultrasonic vibrator 45 through the first electrical wiring 48. Accordingly, the ultrasonic vibrator 45 generates ultrasonic vibration.

The ultrasonic vibrator 45 is attached to the horn member 46. The horn member 46 is made of a metallic material. The horn member 46 is provided with a cross-sectional area variable portion that has an essentially circular-cone shape, and the cross-sectional area of which becomes narrower towards the distal end direction of the probe 36. The ultrasonic vibration generated at the ultrasonic vibrator 45 is transmitted to the horn member 46, and an amplitude of the ultrasonic vibration is enlarged by the cross-sectional area variable portion.

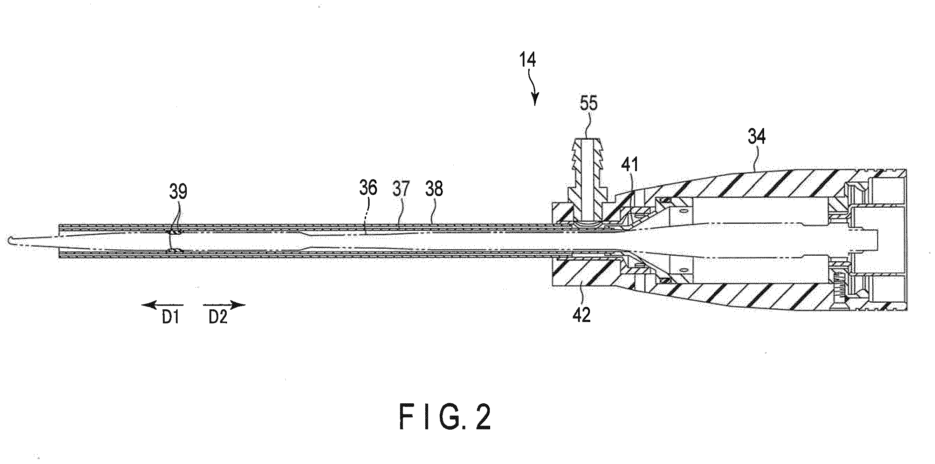

The probe 36 is made of, for example, a conductive metal material (e.g., a titanium alloy) as a rod-like shape. The probe 36 has a shaft portion 52 extending as a rod-like shape. As shown in the example of FIG. 4, which is useful for the understanding of the present invention, the probe 36 is curved as an arch shape to be warped in one direction at the distal end portion. The shaft portion 52 is provided at the distal end side with a treatment portion 53 (excision blade) projecting as a rake-like shape (hook-like shape) in a direction crossing a direction in which the shaft portion 52 extends, and a back surface 54 provided opposite to the treatment portion 53. As shown in FIG. 3, the proximal end of the shaft portion 52 of the probe 36 is concatenated with the horn member 46. Accordingly, the probe 36 transmits ultrasonic vibration generated by the ultrasonic vibrator 45 to perform treatment such as excising a bone (treatment target portion) by the treatment portion 53 of the probe 36. The probe 36, the first sheath 37, and the second sheath 38 are curved at the distal end side in FIG. 4; however, they are depicted as a linear shape in FIG. 2 for simplification.

The power supply unit 15 may be connected to the probe 36 at one side of a second wiring, or connected to the second sheath 38 at another side of the second wiring so that the power supply unit 15 supplies a high-frequency current between the probe 36 and the second sheath 38 to perform bi-polar treatment. In this structure, the probe 36 acts as one electrode for bi-polar treatment. The second sheath 38 acts as another electrode for bi-polar treatment.

As shown in FIG. 4, the first sheath 37 is made, for example, of a metallic material as a cylindrical shape along the outer shape of the probe 36, and the diameter of which becomes gradually smaller toward the distal end. The first sheath 37 has a first edge 37A at the distal end distance D1 side of the probe 36. The inner surface of the first sheath 37 may be covered, for example, with a first coating film made of a resin material having electrical insulation properties and heat insulation properties.

The second sheath 38 is made, for example, of a metallic material having conductive properties as a cylindrical shape along the outer shape of the first sheath 37 (and the probe 36), and the diameter of which becomes gradually smaller toward the distal end. The second sheath 38 has a second edge 38A at the distal end distance D1 side of the probe 36. The thickness of the second sheath 38 is greater than the thickness of the first sheath 37. The outer surface of the cylindrical second sheath 38 may be covered, for example, with a second coating film made of a resin material having electrical insulation properties and heat insulation properties. The first sheath 37 and the second sheath 38 each are curved along the curved shape of the probe 36.

The suction path 28 is formed in a space between the first sheath 37 and the second sheath 38. The suction path 28 has the suction opening 31 externally exposed at the distal end direction D1 side. The suction opening 31 is provided between the first edge 37A and the second edge 38A. The suction path 28 has a connection port 55 (connection part) to be connected to the suction source 17, at the proximal end direction D2 side of the probe 36 (see FIG. 2).

As shown in FIGS. 4 and 5, the height of the treatment portion 53 (excision blade) in the direction crossing the longitudinal axis C of the probe 36 is represented as H1, and the height of the suction path 28 (suction opening 31) in the direction crossing the longitudinal axis C of the probe 36 is represented as H2. In this structure, the suction path 28 (suction opening 31) is formed to satisfy the relationship of H2>H1.

As shown in FIG. 1, the case 34 is provided, for example, with two buttons 43A and 43B. A first button 43A is associated, for example, with a coagulation/excision mode for performing incision or excision of a bone or living tissue while performing blood coagulation (coagulation) to living tissue. A second button 43B is associated, for example, with a coagulation mode for performing blood coagulation, etc. when a bone or living tissue bleeds.

The power supply unit 15 includes the energy output unit 51 and a controller 56 that controls the energy output unit 51. The controller 56 can control power supply from the energy output unit 51. That is, when an operator operates the first button 43A, the controller 56 controls the energy output unit 51 to output power suitable for incision or excision of bone tissue (living tissue). At this time, the treatment portion 53 of the probe 36 ultrasonic-vibrates with a relatively large amplitude. Similarly, when an operator operates the second button 43B, the controller 56 controls the energy output unit 51 to output power suitable for coagulation or blood coagulation of bone tissue (living tissue). At this time, the treatment portion 53 of the probe 36 ultrasonic-vibrates with a relatively small amplitude.

The operation of the treatment system 11 for a joint (the arthroscopic surgery method using the treatment system 11 for a joint) will be described with reference to FIGS. 1 to 5.

An operator inserts the insertion portion 24 of the arthroscope 18 into a joint cavity 26, as shown in FIG. 1. Under the observation by the arthroscope 18, the first sheath 37, the second sheath 38, and the probe 36 of the treatment device 14 are inserted into the joint cavity 26. For insertion of the arthroscope 18 and the treatment device 14, a trocar (tubular guide) allocated beforehand to pierce a skin of a patient can be used. The probe 36 which is ultrasonic vibrated can be used for removing part of joint capsule 26A around the joint cavity 26. The probe 36 that is used for the treatment to the first bone 12 described below can be adopted for this operation, and accordingly, there is no need to exchange the treatment device 14. Prior to the treatment by the treatment device 14, the joint cavity 26 is filled with liquid having conductive properties (liquid including electrolytes) such as a perfusate 16 for arthroscope use including a lactated Ringer's solution or physiological saline by a known method.

As shown in FIG. 1, the first sheath 37, the second sheath 38, and the probe 36 are inserted between the first bone 12 and the second bone 13 opposed to the first bone 12. When the operator operates the first button 43A while the treatment portion 53 of the probe 36 is brought into contact with the first bone 12 that is a treatment target, the probe 36 and the treatment portion 53 are ultrasonically vibrated. The operator can perform treatment such as shaving an undesired part of the first bone 12 to be treated by the probe 36 while finely adjusting the position and the angle of the probe 36. This treatment includes various treatments such as excision of a bone spur of the first bone 12, a cortical bone, a cartilage, and a cancellous bone around the first bone 12, excision of a synovial membrane, excision of semi-lunar cartilage, and removal of other living tissue around the first bone 12, etc.

If fragments of living tissue shaved by ultrasonic vibration, blood leaked from living tissue by the treatment, or foam generated by cavitation due to ultrasonic vibration, etc. are left, they are dispersed around the probe 36 and the perfusate 16 becomes cloudy, thereby obstructing the view through the arthroscope 18. In the present embodiment, the controller 56 outputs ultrasonic energy to the probe 36, and activates the suction source 17 simultaneously. Accordingly, when living tissue is excised or removed, fragments or blood dispersed around the living tissue can be removed by suctioning them from the suction opening 31 together with the perfusate 16. By this processing, it is possible to always ensure a clear view through the arthroscope 18. Since the diameter of living tissue fragments generated by this treatment is essentially equal to or less than the height of the treatment portion 53, the occurrence of clogging of the suction path 28 with living tissue fragments larger than the suction opening 31 can be prevented. During operations, the amount of perfusate 16 reduces due to suction removal. Accordingly, it is preferable that the perfusate 16 is suitably supplied to maintain a suitable amount of perfusate 16.

On the other hand, when the operator performs treatment to tissue including a blood vessel (for example, the first bone 12 or the ambient tissue), and the tissue bleeds, the operator can perform blood coagulation if necessary. When performing blood coagulation, the operator brings the back surface 54 opposed to the treatment portion 53 of the probe 36 shown in FIG. 1, or the surface on which the treatment portion 53 is provided, into contact with the tissue that is bleeding (for example, the first bone 12 or the ambient tissue). In this state, the operator can operate the second button 43 to perform coagulation or blood coagulation to the tissue that is bleeding.

According to the first embodiment, the treatment device 14 includes the probe 36 including the treatment portion 53 that performs treatment to the treatment target portion, ultrasonic vibration being transmitted to the probe 36; the first sheath 37 including the first edge 37A and covering the periphery of the probe 36; the second sheath 38 including the second edge 38A and covering the periphery of the first sheath 37; the suction path 28 including the suction opening 31 between the first edge 37A and the second edge 38A and provided between the first sheath 37 and the second sheath 38; and the connection part communicating with the suction path 28 and connected to the suction source 17.

With this structure where the suction path 28 is provided between the first sheath 37 and the second sheath 38, and the suction opening 31 is provided between the first edge 37A and the second edge 38A, fragments of living tissue or blood generated due to the treatment can be retrieved by the suction opening 31 provided in the vicinity of the treatment portion 53. Accordingly, the clear view can be ensured during the operation, thereby improving the safety of the operation and improving operability for the operator. In addition, with the above structure, the diameter of the suction path 28 can be defined as greater in comparison with the structure where the suction path 28 is provided within the probe 36. Accordingly, the suction path 28 can be prevented from being clogged. The probe 36 is generally made of metal such as titanium or titanium alloy, etc which have touch and high-melting properties. However, titanium etc. is difficult to machine, and may cause difficulty in machining the probe 36 to form the suction path 28. According to the above structure where the suction path 28 is formed independently from the probe 36, it is possible to eliminate difficulty in processing the probe 36.

In this case, if the height of the treatment portion 53 in the direction crossing the longitudinal axis C of the probe 36 is represented as H1, the height of the suction path 28 including the suction opening 31 in the direction crossing the longitudinal axis C of the probe 36 is represented as H2, and the suction path 28 including the suction opening 31 has a portion that satisfies the relationship of H2>H1.

With this structure, the diameter of living tissue fragments generated due to the treatment (excision) can essentially be H1 or less. It is also possible to provide a portion having the height of H2 greater than H1 in the suction path 28. Accordingly, it is possible to prevent clogging by living tissue fragments at the portion having the height of H2, and the operator can perform the treatment safely and smoothly.

The thickness of the first sheath 37 is smaller than the thickness of the second sheath 38. With this structure, the second sheath 38 can maintain a certain degree of strength, and even if an external impulse is applied to the second sheath 38 which is externally provided, the risk that the second sheath 38 is dented can be reduced. On the other hand, the first sheath 37 can reduce the thickness, thereby reliably increasing the diameter (cross-sectional area) of the suction path 28. Thus, the risk that the suction path 28 is clogged can be reduced.

The first sheath 37 and the second sheath 38 each are curved along the curved shape of the probe 36. With this structure, the diameter of the first sheath 37 and the second sheath 38 can be reduced to the greatest extent possible at the distal end side of the probe 36. Accordingly, visibility for the probe 36 can be improved, and the accessibility of the probe 36 relative to the treatment target portion can be improved, for example, when inserting the probe 36 into a narrow space between living tissue.

### (Modification of First Example)

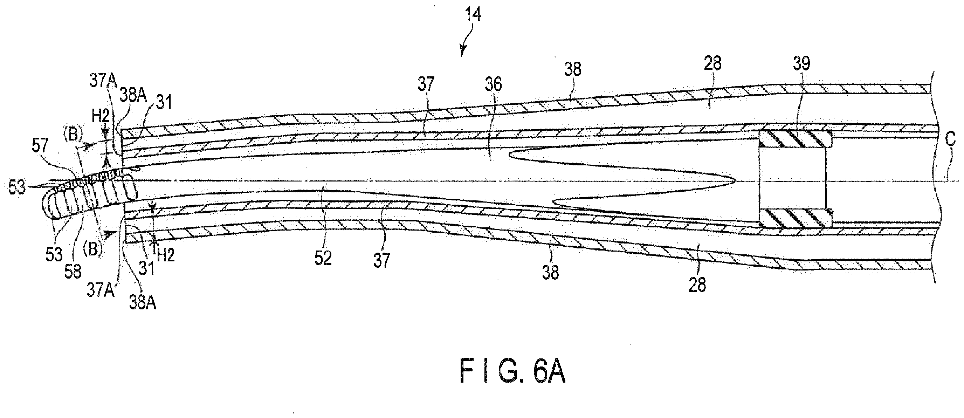

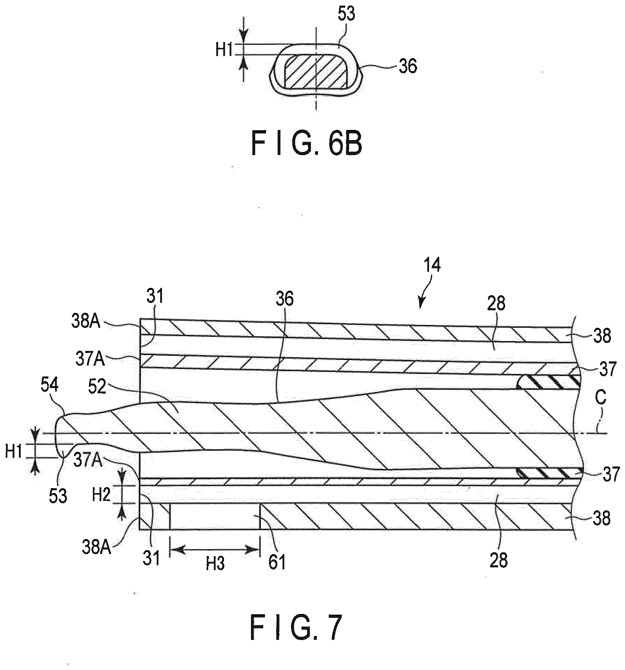

A modification of the treatment system 11 for a joint according to the first example will be described with reference to FIG. 6. In the first modification, the shape of a probe 36 is different from that of the first embodiment; however, the other elements are similar to those of the first embodiment. Accordingly, mainly elements different from the first embodiment or the first example will be explained, and the elements similar to the first embodiment or the first example will not be explained or shown in the drawings.

Unlike the first embodiment and the first example, the probe 36 is formed into an overall rod-like shape. The probe 36 is curved as an arch shape to be warped in one direction at the distal end portion. A plurality of treatment portions 53 (excision blades) are provided both on a primary treatment surface 57 and probe side surfaces 58 adjacent to both sides of the treatment surface 57, instead of being formed as a rake-like shape projecting in a particular direction, as in the first embodiment and in the first example.

A first sheath 37 is made, for example, of a metallic material as a cylindrical shape along the outer shape of the probe 36, and the diameter of which becomes gradually smaller toward the distal end. The inner surface of the first sheath 37 may be covered, for example, with a first coating film made of a resin material having insulation properties and heat insulation properties.

A second sheath 38 is made, for example, of a metallic material as a cylindrical shape along the outer shape of the first sheath 37, and the diameter of which becomes gradually smaller toward the distal end. The thickness of the second sheath 38 is greater than the thickness of the first sheath 37. The outer surface of the cylindrical second sheath 38 may be covered, for example, with a second coating film made of a resin material having insulation properties and heat insulation properties. The first sheath 37 and the second sheath 38 each are curved along the curved shape of the probe 36.

The suction path 28 is formed in a space between the first sheath 37 and the second sheath 38. The suction path 28 has the suction opening 31 externally exposed at the distal end direction D1 side. The suction opening 31 is provided between the first edge 37A and the second edge 38A. The suction path 28 has a connection port 55 (connection part) to be connected to the suction source 17, at the proximal end direction D2 side.

As shown in FIGS. 6A and 6B, the height of the treatment portion 53 (blade height) in the direction crossing the longitudinal axis C of the probe 36 is represented as H1, and the height of the suction path 28 (suction opening 31) in the direction crossing the longitudinal axis C of the probe 36 is represented as H2. In this structure, the suction path 28 (suction opening 31) is formed to satisfy the relationship of H2>H1.

The advantages of the treatment system for a joint of this modification are similar to those of the first embodiment.

### [Second Example]

A treatment system 11 for a joint according to the second example useful to understand the present invention will be described with reference to FIG. 7. The treatment system 11 for a joint of the second example differs from the first embodiment in that an opening portion 61 is provided at the distal end direction D1 side of a second sheath 38; however, the other elements are similar to the first embodiment or the first example. Accordingly, mainly elements different from the first embodiment or first example will be explained, and the elements similar to the first embodiment or the first example will not be explained or shown in the drawings.

A first sheath 37 is made, for example, of a metallic material as a cylindrical shape along the outer shape of the probe 36, and the diameter of which becomes gradually smaller toward the distal end. The inner surface of the first sheath 37 may be covered, for example, with a first coating film made of a resin material having insulation properties and heat insulation properties.

The second sheath 38 is made, for example, of a metallic material as a cylindrical shape along the outer shape of the first sheath 37 (and the probe 36), and the diameter of which becomes gradually smaller toward the distal end. The thickness of the second sheath 38 is greater than the thickness of the first sheath 37. The outer surface of the cylindrical second sheath 38 may be covered, for example, with a second coating film made of a resin material having insulation properties and heat insulation properties.

The opening portion 61 is defined in the vicinity of a second edge 38A of the second sheath 38. The opening portion 61 is a circular through-hole piercing through the second sheath 38 in the thickness direction. In this embodiment, one opening portion 61 is provided to the second sheath 38. The opening portion 61 is provided at the side where a treatment portion 53 of the second sheath 38 is provided (the direction in which the treatment portion 53 projects). The opening portion 61 communicates with a suction path 28.

The suction path 28 is formed in a space between the first sheath 37 and the second sheath 38. A suction opening 31 of the suction path 28 is provided between the first edge 37A and the second edge 38A. The suction path 28 has a connection port 55 (connection part) to be connected to the suction source 17, at the proximal end direction D2 side.

In this case, if the height of the treatment portion 53 in the direction crossing the longitudinal axis C of the probe 36 is represented as H1, and the height of the suction path 28 (suction opening 31) in the direction crossing the longitudinal axis C of the probe 36 is represented as H2, the suction path 28 (suction opening 31) is formed to satisfy the relationship of H2>H1. On the other hand, if the diameter of the opening portion 61 is represented as H3, the opening portion 61 is defined in the second sheath 38 to satisfy the relationship of H3>H2. Accordingly, in this embodiment, the treatment system 11 having the opening portion 61 that can better prevent clogging in comparison with providing a suction opening 31 is realized.

The operation of the treatment system 11 for a joint according to the present embodiment will be described with reference to FIG. 7.

An operator inserts the insertion portion 24 of the arthroscope 18 into a joint cavity 26, as shown in FIG. 1. Under observation with the arthroscope 18, the first sheath 37, the second sheath 38, and the probe 36 of the treatment device 14 are inserted into the joint cavity 26. A trocar is used for inserting the arthroscope 18 and the treatment device 14. Prior to the treatment by the treatment device 14, the joint cavity 26 is filled with liquid having conductive properties (liquid including electrolyte) such as perfusate 16 for arthroscope use including a lactated Ringer's solution or physiological saline by a known method.

As shown in FIG. 1, the first sheath 37, the second sheath 38, and the probe 36 are inserted between the first bone 12 and the second bone 13 opposed to the first bone 12. When the operator operates a first button 43 while the treatment portion 53 of the probe 36 is brought into contact with a first bone 12 which is a treatment target, ultrasonic energy can be applied to the probe 36. By this operation, the probe 36 and the treatment portion 53 at the distal end thereof are ultrasonic-vibrated. The operator can perform treatment such as shaving undesirable parts of the first bone 12 to be treated by the probe 36 while finely adjusting the position and angle of the probe 36.

If fragments of living tissue shaved by ultrasonic vibration, blood, or foam, etc. generated by cavitation are left, they are dispersed around the probe 36 and the perfusate 16 becomes cloudy, thereby obstructing the view through the arthroscope 18. Similar to the present embodiment, a controller 56 outputs ultrasonic energy to the probe 36, and activates a suction source 17 simultaneously. Accordingly, when living tissue is excised or removed, fragments or blood dispersed around the living tissue can be removed by suctioning them from the opening portion 61 and the suction opening 31 together with the perfusate 16. In the present embodiment, the opening portion 61 is provided at the side where the treatment portion 53 is provided, and accordingly, living tissue fragments are efficiently suctioned in the vicinity of the place where the living tissue fragments are generated. By this processing, it is possible to always ensure a clear view through the arthroscope 18. Since the diameter of living tissue fragments generated by this treatment is essentially equal to or less than the height of the treatment portion 53, the occurrence of clogging of the suction path 28 with living tissue fragments larger than the suction opening 31 can be prevented. Even if the suction opening 31 is clogged, living tissue fragments or blood can be suctioned together with the perfusate 16 through the opening portion 61.

On the other hand, when the operator performs treatment to tissue including a blood vessel (for example, the first bone 12 or the ambient tissue), and the tissue bleeds, the operator can operate a second button 43 to supply ultrasonic energy suitable for coagulation or blood coagulation to the probe 36 to perform blood coagulation.

According to the present embodiment, the second sheath 38 has at least one opening portion 61 that is provided in the vicinity of the second edge 38A and communicates with the suction path 28. With this structure, the treatment device 14 can suction living tissue fragments, etc. not only through the suction opening 31 of the suction path 28, but also through the opening portion 61. Thus, it is possible to clear the view through the arthroscope 18, and to improve the safety of operation and operability for the operator.

The opening portion 61 is provided at the side where a treatment portion 53 is provided. With this structure, living tissue fragments generated due to the treatment by the treatment portion 53 can be retrieved at the position close to the treatment portion 53 through the opening portion 61. By this processing, it is possible to prevent the perfusate 16 from becoming cloudy, and to always ensure a clear view.

### (First modification of second example)

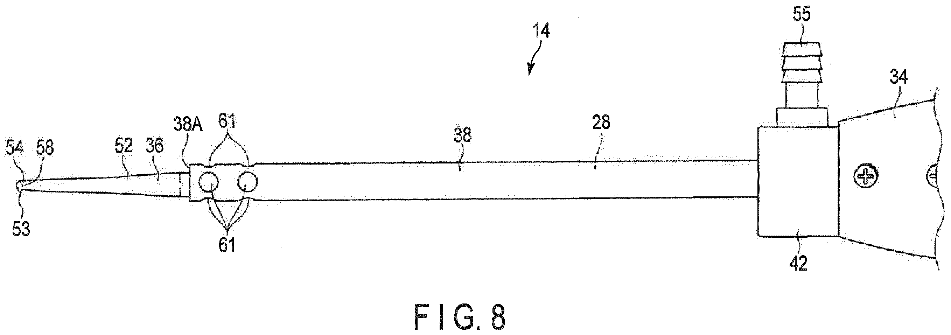

The first modification of the treatment system 11 for a joint according to the second example will be described with reference to FIG. 8. In the first modification, the number of opening portions 61 provided in the second sheath 38 is different from that of first embodiment or the second example; however, the other elements are similar to those of the second example. Accordingly, mainly elements different from the first embodiment or second example will be explained, and the elements similar to the second example will not be explained or shown in the drawings.

As shown in FIG. 1, the probe 36 is made, for example, of a metal material (e.g., a titanium alloy) into a rod-like shape. The probe 36 includes a shaft portion 52, a treatment portion 53 (excision blade) provided at the distal end side of the shaft portion 52 and projecting as a rake-like shape (hook-like shape) in the direction crossing the direction in which the shaft portion 52 extends, a back surface 54 provided opposite to the treatment portion 53, and a pair of probe side surfaces 58 provided consecutively to the treatment portion 53 and the back surface 54. The second sheath 38 is provided with a plurality of opening portions 61 in the vicinity of a second edge 38A provided at the distal end. Each of the opening portions 61 is a circular through-hole piercing through the second sheath 38 in the thickness direction. Two of the opening portions 61 are provided at the side where the treatment portion 53 of the probe 36 is provided (the direction in which the treatment portion 53 projects). Two of the plurality of the opening portions 61 are provided at the side where the back surface 54 of the probe 36 is provided. Two of the plurality of the opening portions 61 are provided at the side where one of the probe side surfaces 58 of the probe 36 is provided, and two of the plurality of the opening portions 61 are provided at the side where the other one of the probe side surfaces 58 of the probe 36 is provided. Each of the opening portions 61 communicates with a suction path 28.

The suction path 28 is formed in a space between the first sheath 37 and the second sheath 38. The suction path 28 has the suction opening 31 externally exposed at the distal end side of the longitudinal axis C of the probe 36. The suction path 28 has a connection port 55 (connection part) to be connected to the suction source 17, at the proximal end direction D2 side.

In this case, if the height of the treatment portion 53 in the direction crossing the longitudinal axis C of the probe 36 is represented as H1, and the height of the suction path 28 (suction opening 31) in the direction crossing the longitudinal axis C of the probe 36 is represented as H2, the suction path 28 (suction opening 31) is formed to satisfy the relationship of H2>H1, in the manner similar to the second embodiment. If the diameter of the opening portion 61 is represented as H3, each of the opening portions 61 is defined in the second sheath 38 to satisfy the relationship of H3>H2, in the manner similar to the second example.

The operation of the treatment system 11 for a joint (the arthroscopic surgery method using the treatment system for a joint) according to the present modification will be described with reference to FIG. 8, for example.

An operator inserts the first sheath 37, the second sheath 38 and the probe 36 between a first bone 12 and a second bone 13 opposed to the first bone 12, as shown in FIG. 1. When the operator operates a first button 43 while the treatment portion 53 of the probe 36 is brought into contact with the first bone 12 which is a treatment target, ultrasonic energy can be applied to the probe 36. By this operation, the probe 36 and the treatment portion 53 at the distal end thereof are ultrasonic-vibrated. The operator can perform treatment such as shaving an undesired part of the first bone 12 to be treated by the probe 36 while finely adjusting the position and the angle of the probe 36.

Fragments of living tissue shaved by ultrasonic vibration or blood are dispersed around the probe 36 and the perfusate 16 becomes cloudy, thereby obstructing the view through the arthroscope 18. In the present modification, the controller 56 outputs ultrasonic energy to the probe 36, and activates the suction source 17 simultaneously. Accordingly, when living tissue is excised or removed, fragments or blood dispersed around the probe 36 are removed by suctioning them from the suction opening 31 of the suction path 28 and the opening portions 61 together with the perfusate 16. In this modification, with the structure where a plurality of opening portions 61 are provided, living tissue fragments are efficiently suctioned and removed. By this processing, it is possible to always ensure a clear view through the arthroscope 18.

According to the modification, the treatment device 14 can suction living tissue fragments, etc. not only through the suction opening 31 of the suction path 28, but also through the opening portions 61; therefore, the view through the arthroscope 18 is cleared, and the safety of operation and the operability for the operator can be improved.

### (Second modification of second example)

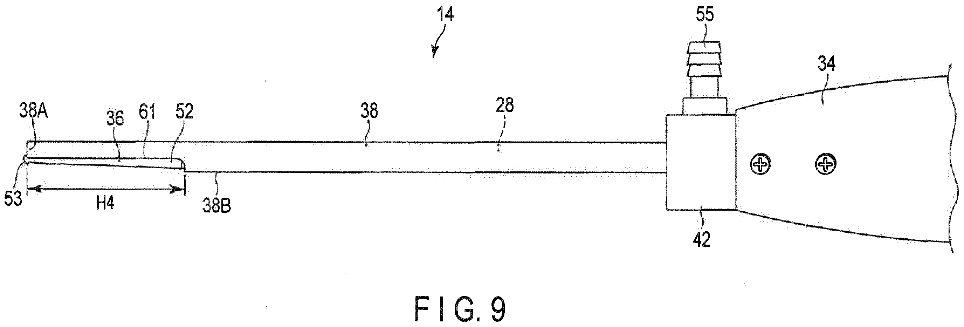

The second modification of the treatment system for a joint according to the second example will be described with reference to FIG. 9. The second modification is different from the second example in that an opening portion 61 is formed as a cut-out portion; however, the other elements are similar to those of the second example. Accordingly, mainly elements different from the second example will be explained, and the elements similar to the second example will not be explained or shown in the drawings.

The second sheath 38 has an opening portion 61 defined in the vicinity of the distal end of the probe 36. The opening portion 61 is defined as a cut-out portion spanning from a side surface 38B (outer surface) to a second edge 38A of the second sheath 38. The opening portion 61 is provided at the side where a treatment portion 53 of the second sheath 38 is provided (the direction in which the treatment portion 53 projects). The opening portion 61 communicates with a suction path 28.

The suction path 28 is formed in a space between the first sheath 37 and the second sheath 38. The suction path 28 has the suction opening 31 exposed to the external at the distal end direction D1 side. The suction path 28 has a connection port 55 (connection part) to be connected to the suction source 17, at the proximal end direction D2 side.

In this modification, if the length of the opening portion 61 along the longitudinal axis C of the probe 36 is represented as H4, H4 is far greater than the height H2 of the suction path 28 (see FIG. 7) in the direction crossing the longitudinal axis C of the probe 36. Accordingly, in this example, the treatment system 11 having the opening portion 61 that can better prevent clogging in comparison with providing a suction opening 31 is realized.

According to the modification, the advantages essentially similar to the second embodiment can be realized. The length H4 of the opening portion 61 is far greater than the diameter of fragments (living tissue fragments) generated by the treatment portion 53; thus, it is possible to prevent the suction path 28 from being clogged with the living tissue fragments to the greatest extent possible. In addition, since the opening portion 61, which is a cut-out portion, is provided at the side where the treatment portion 53 is provided, the living tissue fragments can be efficiently suctioned and removed.

### [Second Embodiment]

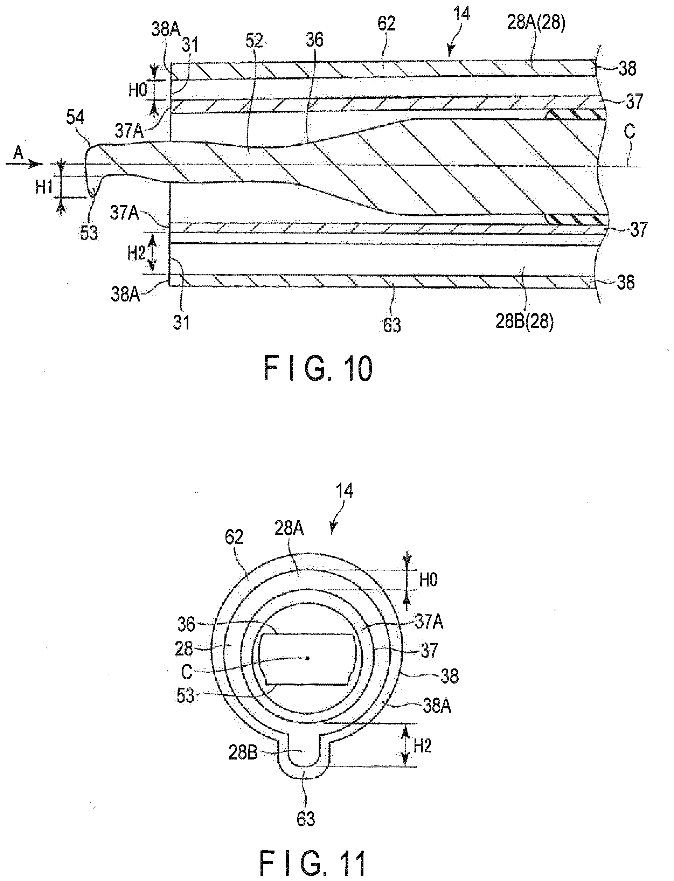

A treatment system for a joint according to the second embodiment will be described with reference to FIGS. 10 and 11. The treatment system 11 for a joint of the second embodiment adopts a second sheath 38 having a different shape from that of the first embodiment; however, the other elements are similar to the first embodiment. Accordingly, mainly elements different from the first embodiment will be explained, and the elements similar to the first embodiment will not be explained or shown in the drawings.
A first sheath 37 is made, for example, of a metallic material as a cylindrical shape along the outer shape of the probe 36, and the diameter of which becomes gradually smaller toward the distal end.

The second sheath 38 is made, for example, of a metallic material as a cylindrical shape along the outer shape of the first sheath 37 (and the probe 36), and the diameter of which becomes gradually smaller toward the distal end. The thickness of the second sheath 38 is greater than the thickness of the first sheath 37. As shown in FIG. 11, the cross-section of the second sheath 38 is a keyhole-like shape. The second sheath 38 includes a second sheath main body 62 having a cylindrical shape, and a protrusion 63 protruding toward the side where the treatment portion 53 is provided from the second sheath main body 62. The protrusion 63 protrudes from the second sheath main body 62 as a half-round cross sectional shape. The protrusion 63 consecutively extends along the longitudinal axis C of the probe 36.

As shown in FIG. 10, the suction path 28 is formed in a space between the first sheath 37 and the second sheath 38. The suction path 28 has a suction opening 31 externally exposed at the distal end direction D1 side. A suction opening 31 is provided between a first edge 37A of the first sheath 37 and the second edge 38A of the second sheath 38. The suction path 28 has a connection port 55 (connection part) to be connected to the suction source 17, at the proximal end direction D2 side.

As shown in FIGS. 10 and 11, the suction path 28 includes a first part 28A which corresponds to the inside of the second sheath main body 62, and a second part 28B which corresponds to the inside of the protrusion 63. The height of the treatment portion 53 in the direction crossing the longitudinal axis C of the probe 36 is represented as H1, the height of the first part 28A in the direction crossing the longitudinal axis C of the probe 36 is represented as H0, and the height of the second part 28B in the direction crossing the longitudinal axis C of the probe 36 is represented as H2. In this case, the suction path 28 (suction opening 31) is formed to satisfy the relationship of H2>H1>H0.

The operation of the treatment system 11 for a joint (the arthroscopic surgery method using the treatment system for a joint) according to the present embodiment will be described with reference to FIGS. 10 and 11, for example.

An operator inserts the first sheath 37, the second sheath 38 and the probe 36 between a first bone 12 and a second bone 13 opposed to the first bone 12, as shown in FIG. 1. When the operator operates a first button 43 while the treatment portion 53 of the probe 36 is brought into contact with the first bone 12 which is a treatment target, ultrasonic energy can be applied to the probe 36. By this operation, the probe 36 and the treatment portion 53 at the distal end thereof are ultrasonically vibrated. The operator can perform treatment such as shaving an undesired part of the first bone 12 to be treated by the probe 36 while finely adjusting the position and angle of the probe 36.

Fragments of living tissue shaved by ultrasonic vibration or blood are dispersed around the probe 36 and the perfusate 16 becomes cloudy, thereby obstructing the view through the arthroscope 18. On the other hand, the controller 56 outputs ultrasonic energy to the probe 36, and activates the suction source 17 simultaneously. Accordingly, when living tissue is excised or removed, fragments or blood dispersed around the living tissue can be removed by suctioning them from the suction opening 31 of the suction path 28 together with the perfusate 16. In this case, the suction removal is performed through the second part 28B that has a greater height in the direction crossing the longitudinal axis C, and accordingly, it is possible to prevent the suction path 28 from being clogged to the great extent possible, and to always ensure a clear view through the arthroscope 18.

According to the present embodiment, the second sheath 38 has the protrusion 63 protruding along the direction where the treatment portion 53 projects, and the suction path 28 satisfies the relationship of H2>H1 at the portion corresponding to the protrusion 63.

With this structure, the protrusion 63 reliably has a portion that satisfies the relationship of H2>H1, and accordingly, clogging of living tissue fragments is prevented at the portion corresponding to the protrusion 63 in the suction path 28. Accordingly, a clear view can be ensured during the operation, thereby improving the safety of the operation and improving operability of the operator. In addition, the part having the greater diameter in the second sheath 38 can be minimized. With this structure, it is possible to prevent reducing the visibility of the probe 36 during the operation, and to improve accessibility when the probe 36 is inserted into a narrow space between living tissue.

### [Third Embodiment]

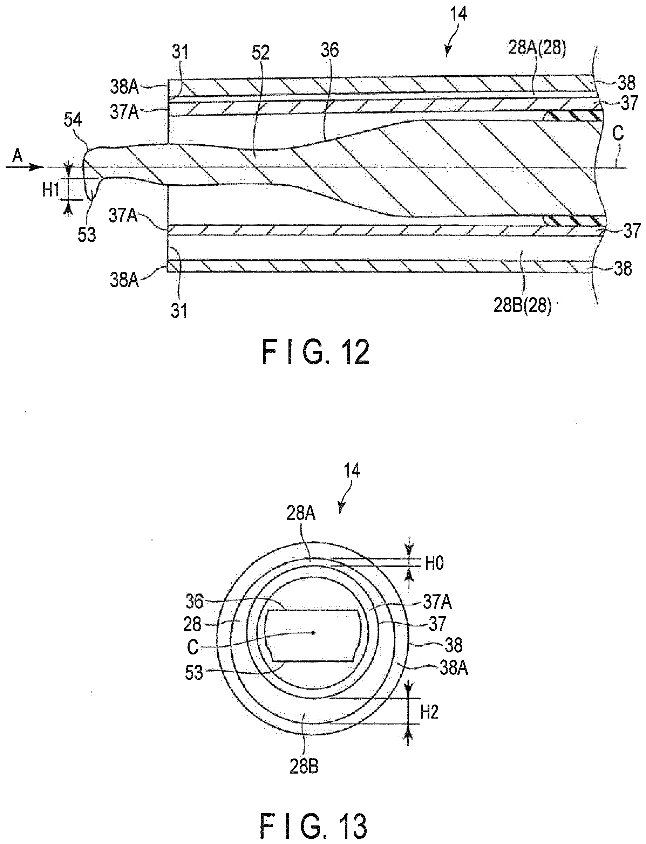

A treatment system for a joint according to the third embodiment will be described with reference to FIGS. 12 and 13. The treatment system 11 for a joint of the third embodiment is provided with a second sheath 38 at a different position from that of the first embodiment; however, the other elements are similar to the first embodiment. Accordingly, mainly elements different from the first embodiment will be explained, and the elements similar to the first embodiment will not be explained or shown in the drawings.

A first sheath 37 is made, for example, of a metallic material as a cylindrical shape along the outer shape of a probe 36, and the diameter of which becomes gradually smaller toward the distal end.

The second sheath 38 is made, for example, of a metallic material as a cylindrical shape along the outer shape of the first sheath 37 (and the probe 36), and the diameter of which becomes gradually smaller toward the distal end. The thickness of the second sheath 38 is greater than the thickness of the first sheath 37. The second sheath 38 is provided to be eccentric from the central axis C (longitudinal axis) of the probe 36 toward the side where a treatment portion 53 is provided (the direction in which the treatment portion 53 projects). A treatment device 14 has an adapter 41 that supports the second sheath 38 so that the second sheath 38 is arranged eccentrically as mentioned above (see FIG. 2). The adapter 41 is attached to a position different from the first embodiment. The adapter 41 is arranged eccentric from the central axis C (longitudinal axis) of the probe 36 toward the side where a treatment portion 53 is provided (the direction in which the treatment portion 53 projects). The adapter 41 is detachably attached to a case 34.

A suction path 28 is formed in a space between the first sheath 37 and the second sheath 38. The suction path 28 has a suction opening 31 externally exposed at the distal end side of the longitudinal axis C of the probe 36. The suction opening 31 is provided between a first edge 37A of the first sheath 37 and a second edge 38A of the second sheath 38. The suction path 28 has a connection port 55 (connection part) to be connected to the suction source 17, at the proximal end direction D2 side.

The suction path 28 has a first part 28A (narrow part), the height of which relative to the direction crossing the longitudinal axis C of the probe 36 is small, and a second part 28B (wide part), the height of which relative to the direction crossing the longitudinal axis C of the probe 36 is large. The second part 28B corresponds to the side where the treatment portion 53 is provided (the direction in which the treatment portion 53 projects). The first part 28A corresponds to a back surface 54 side of the probe 36. The height of the treatment portion 53 in the direction crossing the longitudinal axis C of the probe 36 is represented as H1, the height of the first part 28A in the direction crossing the longitudinal axis C of the probe 36 is represented as H0, and the height of the second part 28B in the direction crossing the longitudinal axis C of the probe 36 is represented as H2 (FIGS. 12 and 13). In this case, the suction path 28 (suction opening 31) is formed to satisfy the relationship of H2>H1>H0.

The operation of the treatment system 13 for a joint (the arthroscopic surgery method using the treatment system for a joint) according to the present embodiment will be described with reference to FIGS. 12 and 13, for example.

An operator inserts the first sheath 37, the second sheath 38, and the probe 36 between a first bone 12 and a second bone 13 opposed to the first bone 12, as shown in FIG. 1. When the operator operates a first button 43 while the treatment portion 53 of the probe 36 is brought into contact with the first bone 12 that is a treatment target, ultrasonic energy can be applied to the probe 36. By this operation, the probe 36 and the treatment portion 53 at the distal end thereof are ultrasonically vibrated. The operator can perform treatment such as shaving an undesired part of the first bone 12 to be treated by the probe 36 while finely adjusting the position and angle of the probe 36.

Fragments of living tissue shaved by ultrasonic vibration or blood are dispersed around the probe 36 and the perfusate 16 becomes cloudy, thereby obstructing the view through the arthroscope 18. On the other hand, the controller 56 outputs ultrasonic energy to the probe 36, and activates the suction source 17 simultaneously. Accordingly, when living tissue is excised or removed, fragments or blood dispersed around the living tissue can be removed by suctioning them from the suction opening 31 of the suction path 28 together with the perfusate 16. In this case, the suction removal is performed through the second part 28B that has a greater height in the direction crossing the longitudinal axis C, and accordingly, it is possible to prevent the suction path 28 from being clogged to the great extent possible, and to always ensure a clear view through the arthroscope 18.

According to the present embodiment, the second sheath 38 is arranged eccentric from the central axis C of the probe 36 toward the side where the treatment portion 53 is provided, and the suction path 28 satisfies the relationship of H2>H1 at the portion corresponding to the side where the treatment portion 53 is provided.

With this structure, the portion that satisfies the relationship of H2>H1 is reliably provided at the portion corresponding to the side where the treatment portion 53 is provided (the second part 28B of the suction path 28), and accordingly, clogging of living tissue fragments is prevented at the portion corresponding to the side where the treatment portion 53 is provided. Accordingly, the clear view can be ensured during the operation, thereby improving the safety of the operation and improving workability of the operator. In addition, the second sheath 38 can be formed as a cylindrical shape which is an easy and simple shape. Thus, it is possible to easily ensure the strength of the second sheath 38, and to reduce the manufacturing cost of the treatment device 14.

The present invention is not limited to the above-described embodiments, and can be modified as appropriate in practice without departing from the scope of the invention, as defined by the claims.

It is of course possible to form a treatment system 11 by combining the joint treatment system 11 according to each embodiment.

### REFERENCE SIGNS LIST

11: Treatment system, 14: Treatment device, 17: Suction source, 28: Suction path, 31: Suction opening, 36: Probe, 37: First sheath, 37A: First edge, 38: Second sheath, 38A: Second edge, 38B: Side surface, 53: Treatment portion, 55: Connection port, 61: Opening, 63: Protrusion

## Claims

1. A treatment device (14) comprising:
a probe (36) including an axial portion (52) that defines a longitudinal axis (C) in which ultrasonic vibration is transmitted, and a cutting blade (53) provided on a distal side of the axial portion (52) and projecting in a direction crossing the longitudinal axis (C), the cutting blade (53) being capable of cutting bones and cartilages at a distal end of a projecting direction;
a first sheath (37) enclosing an outer peripheral surface of the axial portion (52) in the probe (36);
a second sheath (38) being arranged eccentric from the longitudinal axis (C) of the probe (36) toward a side where the cutting blade (53) is provided, and enclosing a periphery of the first sheath (37), and the second sheath (38) forming a suction path (28) including, between a periphery of the first sheath (37) and the second sheath (38), a wide portion (28B) corresponding to a side where the cutting blade (53) is provided and a narrow portion (28A) corresponding to a side opposite to the side where the cutting blade (53) is provided;
a connection part (55) communicating with the suction path (28) and connected to a suction source (17),
wherein when a height of the cutting blade (53) in a direction crossing the longitudinal axis (C) direction is represented as H1, a height of the wide portion (28B) in the direction crossing the longitudinal axis (C) is represented as H2, and a height of the narrow portion (28A) in the direction crossing the longitudinal axis (C) is represented as H0, a relationship of H2>H1>H0 is satisfied.

2. The treatment device according to claim 1, wherein a thickness of the first sheath (37) is smaller than a thickness of the second sheath (38).

3. The treatment device according to claim 1, wherein a distal side of the second sheath (38) has at least one opening (31) communicating with the suction path (28).

4. The treatment device according to claim 3, wherein the opening (31) is formed at a side where the cutting blade (53) is provided.

5. The treatment device according to claim 4, wherein when a diameter of the opening (61) is represented as H3, a relationship of H3>H2 is satisfied.

6. A treatment system (11) comprising:
the treatment device according to claim 1; and
the suction source (17) provided to be connected to the connection part (55).

7. The treatment device according to claim 1, wherein a relationship of H2 > H1 > H0 > 0 is satisfied.

## Patentansprüche

1. Behandlungsvorrichtung (14), die umfasst:
eine Sonde (36) mit einem axialen Abschnitt (52), der eine Längsachse (C) definiert, in der eine Ultraschallschwingung übertragen wird, und einer Schneidklinge (53), die an einer distalen Seite des axialen Abschnitts (52) vorgesehen ist und in einer die Längsachse (C) kreuzenden Richtung vorsteht, wobei die Schneidklinge (53) dazu in der Lage ist, Knochen und Knorpel an einem distalen Ende einer Vorstehrichtung zu schneiden;
eine erste Hülse (37), die eine Außenumfangsfläche des axialen Abschnitts (52) in der Sonde (36) umschließt;
eine zweite Hülse (38), die exzentrisch von der Längsachse (C) der Sonde (36) zu einer Seite hin angeordnet ist, an der die Schneidklinge (53) vorgesehen ist, und die einen Umfang der ersten Hülse (37) umschließt, wobei die zweite Hülse (38) einen Saugpfad (28) bildet, der zwischen einem Umfang der ersten Hülse (37) und der zweiten Hülse (38) einen weiten Abschnitt (28B), der einer Seite entspricht, auf der die Schneidklinge (53) vorgesehen ist, und einen schmalen Abschnitt (28A) umfasst, der einer Seite entspricht, die der Seite entgegengesetzt ist, auf der die Schneidklinge (53) vorgesehen ist;
ein Verbindungsteil (55), das mit dem Saugpfad (28) kommuniziert und mit einer Saugquelle (17) verbunden ist,
wobei, wenn eine Höhe der Schneidklinge (53) in einer Richtung, die die Richtung der Längsachse (C) kreuzt, als H1 bezeichnet wird, eine Höhe des weiten Abschnitts (28B) in der Richtung, die die Längsachse (C) kreuzt, als H2 bezeichnet wird und eine Höhe des schmalen Abschnitts (28A) in der Richtung, die die Längsachse (C) kreuzt, als H0 bezeichnet wird, eine Beziehung H2>H1>H0 erfüllt ist.

2. Behandlungsvorrichtung nach Anspruch 1, wobei eine Dicke der ersten Hülse (37) kleiner ist als eine Dicke der zweiten Hülse (38).

3. Behandlungsvorrichtung nach Anspruch 1, wobei eine distale Seite der zweiten Hülse (38) mindestens eine Öffnung (31) aufweist, die mit dem Saugpfad (28) kommuniziert.

4. Behandlungsvorrichtung nach Anspruch 3, wobei die Öffnung (31) an einer Seite ausgebildet ist, an der die Schneidklinge (53) vorgesehen ist.

5. Behandlungsvorrichtung nach Anspruch 4, wobei, wenn ein Durchmesser der Öffnung (61) als H3 bezeichnet wird, eine Beziehung H3>H2 erfüllt ist.

6. Behandlungssystem (11), das umfasst:
die Behandlungsvorrichtung nach Anspruch 1; und
die Saugquelle (17), die zur Verbindung mit dem Verbindungsteil (55) vorgesehen ist.

7. Behandlungsvorrichtung nach Anspruch 1, wobei eine Beziehung H2 > H1 > H0 > 0 erfüllt ist.

## Revendications

1. Dispositif de traitement (14) comprenant :
une sonde (36) incluant une partie axiale (52) qui définit un axe longitudinal (C) dans laquelle une vibration ultrasonore est transmise, et une lame coupante (53) prévue sur un côté distal de la partie axiale (52) et se projetant dans une direction coupant l'axe longitudinal (C), la lame coupante (53) étant apte à couper des os et des cartilages à une extrémité distale d'une direction de projection ;
une première gaine (37) enfermant une surface périphérique extérieure de la partie axiale (52) dans la sonde (36) ;
une deuxième gaine (38) étant agencée excentrique par rapport à l'axe longitudinal (C) de la sonde (36) vers un côté où la lame coupante (53) est prévue, et enfermant une périphérie de la première gaine (37), et la deuxième gaine (38) formant un chemin d'aspiration (28) incluant, entre une périphérie de la première gaine (37) et la deuxième gaine (38), une partie large (28B) correspondant à un côté où la lame coupante (53) est prévue et une partie étroite (28A) correspondant à un côté opposé au côté où la lame coupante (53) est prévue ;
une partie de connexion (55) communiquant avec le chemin d'aspiration (28) et connectée à une source d'aspiration (17),
dans lequel, lorsqu'une hauteur de la lame coupante (53) dans une direction coupant la direction de l'axe longitudinal (C) est représentée comme H1, une hauteur de la partie large (28B) dans la direction coupant l'axe longitudinal (C) est représentée comme H2, et une hauteur de la partie étroite (28A) dans la direction coupant l'axe longitudinal (C) est représentée comme H0, une relation de H2>H1>H0 est satisfaite.

2. Dispositif de traitement selon la revendication 1, dans lequel une épaisseur de la première gaine (37) est plus petite qu'une épaisseur de la deuxième gaine (38).

3. Dispositif de traitement selon la revendication 1, dans lequel un côté distal de la deuxième gaine (38) a au moins une ouverture (31) communiquant avec le chemin d'aspiration (28).

4. Dispositif de traitement selon la revendication 3, dans lequel l'ouverture (31) est formée sur un côté où la lame coupante (53) est prévue.

5. Dispositif de traitement selon la revendication 4, dans lequel, lorsqu'un diamètre de l'ouverture (31) est représenté comme H3, une relation de H3>H2.

6. Système de traitement (11) comprenant :
le dispositif de traitement selon la revendication 1 ; et
la source d'aspiration (17) prévue pour être connectée à la partie de connexion (55).

7. Dispositif de traitement selon la revendication 1, dans lequel une relation de H2 > H1 > H0 > 0 est satisfaite.
